(19) Europäisches Patentamt
European Patent Office
Office européen des brevets

(11) Veröffentlichungsnummer: **0 344 468 A1**

(12) # EUROPÄISCHE PATENTANMELDUNG

(21) Anmeldenummer: 89107725.7

(22) Anmeldetag: 28.04.89

(51) Int. Cl.4: C07D 233/61 , C08G 18/58 , C08G 18/80 , C08G 59/40 , C08G 59/68

Patentanspruch für folgenden Vertragsstaat: ES

(30) Priorität: 28.05.88 DE 3818214

(43) Veröffentlichungstag der Anmeldung: 06.12.89 Patentblatt 89/49

(84) Benannte Vertragsstaaten: AT BE CH DE ES FR GB IT LI NL SE

(71) Anmelder: SCHERING AKTIENGESELLSCHAFT
Berlin und Bergkamen
Waldstrasse 14 Postfach 15 40
D-4709 Bergkamen(DE)

(72) Erfinder: Burba, Christian, Dr. Dipl.-Chem.
Gerhart-Hauptmann-Strasse 9
D-4715 Ascheberg-Herbern(DE)
Erfinder: Mrotzek, Werner, Dr. Dipl.-Ing.
Dresdener Strasse 24
D-4600 Dortmund 1(DE)

(54) **Imidazolyl-Harnstoffverbindungen und deren Verwendung als Härtungsbeschleuniger in Epoxidharzzusammensetzungen zur Herstellung von Formkörpern.**

(57) Gegenstand der Erfindung sind Imidazolyl-Harnstoffverbindungen und deren Verwendung als Härtungsbeschleuniger in Epoxidharzzusammensetzungen, bestehend aus a) einem Epoxidharz, b) Dicyandiamid und gegebenenfalls c) Lösungsmitteln, Füllstoffen, Verstärkungs- oder Einlagematerialien, Pigmenten oder Hilfsstoffen, zur Herstellung von Formkörpern.

EP 0 344 468 A1

# Imidazolyl-Harnstoffverbindungen und deren Verwendung als Härtungsbeschleuniger in Epoxidharzzusammensetzungen zur Herstellung von Formkörpern

Gegenstand der Erfindung sind Imidazolyl-Harnstoffverbindungen und deren Verwendung als Härtungsbeschleuniger in Epoxidharzzusammensetzungen, bestehend aus a) einem Epoxidharz, b) Dicyandiamid und gegebenenfalls c) Lösungsmitteln, Füllstoffen, Verstärkungs- oder Einlagematerialien, Pigmenten oder Hilfsstoffen, zur Herstellung von Formkörpern.

Für die Herstellung von Verbundwerkstoffen werden heute im Prinzip zwei Verfahren angewandt: Das Naß-in-Naß-Verfahren, bei welchem die Verstärkungsmaterialien mit der härtbaren Mischung imprägniert, in feuchtem Zustand unter Formgebung aufeinandergelegt und in der Hitze in einer Stufe bis zum duromeren Endzustand ausgehärtet werden.

Beim Zweistufenverfahren werden aus den Verstärkungsmaterialien und der härtbaren Mischung zunächst sogenannte Prepregs hergestellt, die dann in einem zeitlich getrennten 2. Verfahrensschritt zu Fertigteilen weiterverarbeitet werden.

Die Herstellung der Prepregs erfolgt normalerweise in einem kontinuierlichen Prozeß, in dem die Verstärkungsmaterialien durch ein Imprägnierbad aus einer Lösung des einzusetzenden Harz-Härter-Gemisches geleitet werden. Die Steuerung der auf einer bestimmten Trägermaterialbahn aufzubringenden Imprägniermittelmenge erfolgt dabei außer über die Viskosität der Harz-Härter-Lösung über dem Tränkbad nachgeschaltete Abquetschwalzen. Nach dem Imprägnierprozeß wird durch Wärmzufuhr das in der Imprägnierlösung enthaltene Lösungsmittel verdampft und gleichzeitig das Harzsystem vom A- in den B-Zustand überführt. Aus dem mit flüssigem bis stark klebrigem Imprägniermittel getränkten Verstärkungsmaterialien wird je nach Verfahrensbedingungen und verwendetem Harzsystem ein schwach klebriger bis fast trockener Prepreg. Bei diesem Verfahrensschritt ist es wichtig, daß einerseits das Lösunsmittel der Imprägniermischung vollständig entzogen wird, andererseits aber der für die Prepreg-Härtung im 2. Verfahrensschritt notwendige latente Härter noch nicht anspringt, um ein ungewolltes Ausreagieren der imprägnierten Verstärkungsmaterialien zu verhindern.

Die so erhaltenen Prepregs lassen sich als Rollen zwischenlagern und transportieren, ehe sie dem jeweiligen Anwendungsfall entsprechend zurechtgeschnitten und in Bauteildicke übereinander geschichtet werden. Durch gleichzeitige Druck- und Temperatureinwirkung wird der Prepregstapel zu einem hochfesten Formteil ausgehärtet, wobei die noch niedermolekularen, fließfähigen Harze in den hochmolekularen C-Zustand des Duromers übergehen.

Während beim Einstufenverfahren lange offene Zeiten und kurze Aushärtungszeiten bei niedrigen Härtungstemperaturen gefordert werden, kommt beim Zweistufenverfahren als weiteres Kriterium noch eine möglichst lange Lagerstabilität der Prepregs hinzu. Dabei werden Lagerungstemperaturen unterhalb Raumtemperatur von der Praxis immer weniger akzeptiert.

Von Bedeutung ist, daß nach der Herstellung der gebrauchsfertigen härtbaren Mischung die Materialviskosität über einen möglichst langen Zeitraum weitgehend unverändert bleibt.

Dies ist erforderlich für die Erzielung eines konstanten Harzauftrages und eines gleichbleibenden B-Zustandes, da zum einen die Bedingungen der Produktion nicht laufend den sich ändernden Verhältnissen in der härtbaren Mischung angepaßt werden können und zum anderen dadurch auch die physikalischen Eigenschaften des ausgehärteten Endproduktes negativ beeinflußt werden.

Angestrebt wird in der Praxis eine härtbare Mischung, welche im Imprägnierbad auch über längere Zeit viskositätsmäßig konstant bleibt, bei geringen Temepraturen in kurzer Zeit zum B-Zustand reagiert und dann als Prepreg bei Raumtemperatur ohne chemische Veränderungen lange gelagert werden kann. Weiterhin soll die Aushärtung bei möglichst tiefen Temperaturen innerhalb kurzer Zeit erfolgen, das Temperaturmaximum der exothermen Reaktion auch bei größeren Schichtdicken auf einem niedrigen Niveau verbleiben und das physikalische Eigenschaftsniveau der Endprodukte den Erfordernissen der Praxis angepaßt sein, d. h. insbesondere, daß die von der Matrix bestimmten Übergangstemperaturen oberhalb von 140 °C liegen.

Das in härtbaren Mischungen auf Basis von Epoxidharzen seit langem als Härter verwendete Dicyandiamid wird zur Erzielung der angestrebten Eigenschaften daher in der Regel mit CO-Härtungsmitteln und/oder Beschleunigern kombiniert. Aus der Literatur sind auf diesem Gebiet daher eine Vielzahl von Vorschlägen bekannt geworden.

Die Mitverwendung von tertiären Aminen wie Benzyldimethylamin, tertiär/sekundären Aminen wie 2-Methyl-imidazol, 2-Ethyl-4-methyl-imidazol, tertiär/primären Aminen wie 1-Aminoethyl-imidazol allein oder in Mischungen brachte zwar graduelle Verbesserungen, ohne jedoch gravierende Mängel beseitigen zu können.

Der Austausch von Dicyandiamid gegen wasserfreies monomeres Cyanamid, wie in der DE-AS 21 22 955 empfohlen, konnte ebensowenig überzeugen wie die vorgeschlagene Mitverwendung von aliphatischen und cycloaliphatischen primären Monoamine oder primäre und sekundäre Aminogruppen enthaltende Diamine.

Aus der Literatur sind weiterhin eine Vielzahl von Vorschlägen bekannt, welche als Beschleuniger Verbindungen empfehlen, die ein oder zwei Harnstoffgruppen im Molekül enthalten, insbesondere N,N-Dimethylharnstoffverbindungen wie in den US-PSen 3 661 989, 3 717 612 oder Imidozolylharnstoffe wie in der EP-A-193 068, den US-PSen 4 533 715, 4 358 571, 4 335 228 beschrieben.

Die Mitverwendung dieser Verbindungen als Beschleuniger allein oder in Kombination mit Benzyldimethylamin oder C-bzw. N-substituierten Imidazolen brachte zwar Verbesserungen entweder hinsichtlich der Lagerstabilität oder der Härtungscharakteristik oder der physikalischen Endeigenschaften, war aber hinsichtlich des angestrebten Gesamtniveaus noch verbesserungsbedürftig.

Aufgabe der vorliegenden Erfindung war es, unter Vermeidung der Nachteile des Standes der Technik härtbare Mischungen auf Basis von Epoxidverbindungen, latenten Härtungsmitteln und Härtungsbeschleunigern zu finden, welche auch im B-Zustand bei Raumtemperatur über einen langen Zeitraum lagerstabil sind, dabei aber bei relativ niedrigen Temperaturen innerhalb kurzer Zeiten ohne hohe exo therme Temperaturspitzen zum duromeren Endzustand aushärten und eine den Anforderungen der Praxis entsprechende thermische Beständigkeit besitzen.

Diese Aufgabe wird gelöst durch Mitverwendung eines Härtungsbeschleunigers, welcher mindestens zwei unterschiedliche Harnstoffverbindungen im Molekül enthält.

Gegenstand der Erfindung sind Verbindungen der allgemeinen Formel (I),

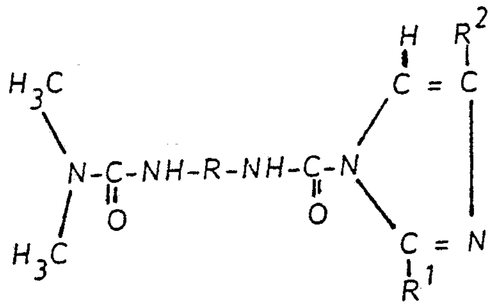

(I)

worin R ein gegebenenfalls substituierter, aliphatischer, cycloatiphatischer, aromatischer, araliphatischer Rest ist und $R^1$ und $R^2$ unabhängig voneinander die Bedeutung H, $-CH_3$, $-C_2H_5$, $-C_6H_5$ haben können.

Ein weiterer Gegenstand der Erfindung ist die Verwendung von Verbindungen der allgemeinen Formel (I),

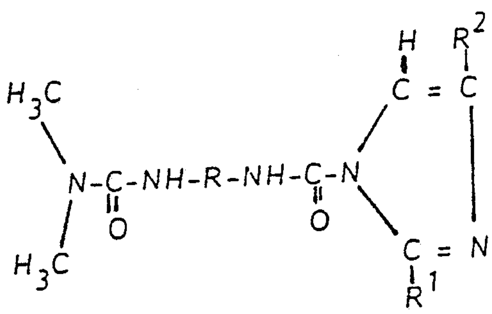

(I)

worin R ein gegebenenfalls substituierter, aliphatischer, cycloaliphatischer, aromatischer, araliphatischer Rest ist und $R^1$ und $R^2$ unabhängig voneinander die Bedeutung H, $-CH_3$, $-C_2H_5$, $-C_6H_5$ haben können als Beschleuniger in härtbaren Mischungen aus:

a) Epoxidharz
b) Dicyandiamid und ggf.
c) Lösungsmitteln, Füllstoffen, Verstärkung- oder Einlagematerialien, Pigmenten, Hilfsstoffen.

Ein weiterer Gegenstand der Erfindung sind härtbare Epoxidharz-Zusammensetzungen enthaltend

a) ein Epoxidharz mit durchschnittlich mehr als einer Epoxidgruppe pro Moleküh!,
b) Dicyandiamid und ggf.
c) Lösungsmittel, Füllstoffe, Verstärkungs- oder Einlagematerialien, Pigmente, Hilfsstoffe und
d) als Härtungsbeschleuniger Verbindungen nach Anspruch 1 bis 4.

Ein weiterer Gegenstand der Erfindung sind härtbare Mischungen gemäß Ansprüchen 6 bis 8, welche dadurch gekennzeichnet sind, daß die Verstärkungsmaterialien bei Raumtemperatur mit dem Bindemittel, bestehend aus
a) Epoxidharz
b) Dicyandiamid
c) Lösungsmittel
d) einem Härtungsbeschleuniger gemäß Anspruch 1 imprägniert und in den halbfesten, aber noch schmelzbaren Zustand (B-Zustand) überführt werden.

Ein weiterer Gegenstand der Erfindung sind Epoxidharzformkörper, welche dadurch gekennzeichnet sind, daß in erster Stufe die Verstärkungsmaterialien bei Raumtemperatur mit dem Bindemittel, bestehend aus
a) Epoxidharz
b) Dicyandiamid
c) Lösungsmittel
d) einem Härtungsbeschleuniger gemäß Anspruch 1 bis 4,
imprägniert und in den halbfesten, aber noch schmelzbaren Zustand (B-Zustand) überführt werden und in zweiter Stufe die so hergestellten Prepregs allein und unter Formgebung oder zwischen zu verklebende Substrate gebracht, unter Anwendung von Druck bei erhöhter Temperatur ausgehärtet werden.

Die weiteren Gegenstände der Erfindung sind durch die Ansprüche gekennzeichnet.

Die erfindungsgemäß mitverwendeten Epoxidharze sind Glycidylester und -ether mit zwei oder mehr Epoxygruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,2 - 0,6, insbesondere die bei Raumtemperatur flüssigen Verbindungen mit Epoxidwerten um 0,45 bis 0,55. Daneben haben sich auch die Glycidylether auf Basis von Bisphenol F und die Novolake als vorteilhaft erwiesen.

Das als Härtungsmittel mitverwendete Dicyandiamid ist ein handelsübliches Produkt und ist unter den bekannten Handelsnamen erhältlich. Die Menge an Dicyandiamid liegt in Abhängigkeit von der eingesetzten Epoxidverbindung im Bereich von 2 bis 10, bei den erfindungsgemäß bevorzugten flüssigen Glycidylethern auf Basis von Bisphenol A im Bereich von 5 bis 10 Gewichtsteilen, bezogen auf 100 Gewichtsteile Diglycidylether.

Die erfindungsgemäß mitverwendeten Härtungsbeschleuniger sind Verbindungen der allemeinen Formel (I),

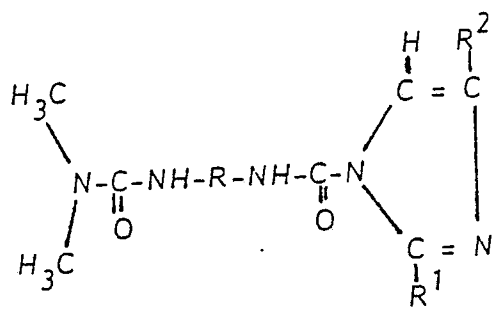

(I)

in welcher R ein gegebenenfalls substituierter aliphatischer, cycloaliphatischer, aromatischer oder araliphatischer Rest ist und $R^1$ und $R^2$ unabhängig voneinander die Bedeutung H, Methyl, Ethyl oder Phenyl haben können.

Diese Verbindungen der allgemeinen Formel (I) werden hergestellt durch Umsetzung der entsprechenden aliphatischen, cycloaliphatischen, aromatischen oder araliphatischen Diisocyanate mit den entsprechenden Imidazolen und Dimethylamin; anstelle der Diisocyanate lassen sich auch deren Urethan- bzw. Harnstoffgruppen enthaltende Prepolymere einsetzen. Im Falle des Isophorondiisocyanates wird durch eine

derartige Vorreaktion mit der Hydroxylverbindung die reaktionsfähigere Isocyanatgruppe bereits weitgehend abgesättigt, so daß für die anschließende Umsetzung mit den Aminkomponenten bevorzugt die reaktionsträgere Isocyanatgruppe in Frage kommt. Die Umsetzung der Isocyanate mit den Imidazolen und dem Dimethylaminen wird bevorzugt in einem Lösungsmittel durchgeführt, das auch Dicyandiamid zu lösen in der Lage ist, so daß man eine gebrauchsfertige Mischung für die Prepregherstellung erhält.

Diese technischen Produkte enthalten daher in statistischer Verteilung noch Anteile der jeweiligen Nebenpro dukte. Mischungen der jeweils reinen Einzelkomponenten sind ebenfalls möglich.

Als Isocyanatkomponenten werden die auf diesem Gebiet üblichen Verbindungen eingesetzt wie:

Isophorondiisocyanat (IPDI), Toluylendiisocyanat (TDI), Diphenylmethandiisocyanat (MDI), Hexamethylendiisocyanat (HDI), Trimethylhexamethylendiisocyanat (TMDI), Xylylendiisocyant (XDI), Tetramethyl-Xylylendiamin (TMXDI) und deren Gemische, sowie Di- u. Trimerisierungsprodukte der entsprechenden Isocyanate.

Neben dem Dimethylamin wird als Imidazol vorzugsweise 2-Methylimidazol und 2-Ethyl-4-methylimidazol eingesetzt.

Die Menge an Härtungsbeschleuniger kann innerhalb weiter Grenzen variiert werden. Sie wird bestimmt durch den beabsichtigten Anwendungszweck und die gegebenenfalls dadurch vorgegebenen Härtungsbedingungen. Erfindungsgemäß werden Mengen im Bereich von 0,1 bis 10, vorzugsweise 1 bis 5 Gewichtsteilen, bezogen auf 100 Gewichtsteile Epoxidverbindung, angewandt.

Zur Modifizierung der Eigenschaften des Endprodukts können neben anderen EP-Harzen auch Modifizierungs- oder Hilfsstoffe mitverwendet werden wie Phenolharze, Melaminharze, Silikonharze, anorganische und organische Füllstoffe wie Quarzmehle, Titandioxid, Ruß, Silikon- oder Butadienkautschuk.

Zur Einstellung der gewünschten Viskosität können unterschiedlich viskose Harze oder (reaktive) Verdünnungsmittel mitverwendet werden, aber auch die üblichen Lösungsmittel wie Dimethylformamid, Aceton, Methylglykol oder deren Mischungen eingesetzt werden.

Zur Herstellung der Prepregs werden organische und anorganische Fasern, Vliese und Gewebe auf Basis von Aramid, Kohlenstoff oder Cellulose, Metallen wir Bor, Stahl, usw. Keramik, insbesondere aber Glas, mitverwendet.

Die Herstellung der Prepregs geschieht nach der an sich bekannten Methode, bei der die Verstärker- bzw. Trägermaterialien in einem Imprägnierbad mit der reaktiven Harzmischung getränkt und nach dem Abquetschen der überschüssigen Harzmenge kontinuierlich unter Energiezufuhr (meist Wärme), bei gleichzeitigem Entzug eventuell vorhandener Lösungsmittel, vom A- in den B-Zustand überführt werden. Je nach gewünschter Prepreg-Konsistenz (klebrig bis fest) werden diese anschließend beidseitig mit einer Trennfolie versehen und für die Lagerung und den Transport aufgerollt. Die Weiterverarbeitung erfolgt durch Zuschneiden und Zusammenlegen der einzelnen Prepreg-Lagen zu einem Stapel, aus dem durch formgebende Maßnahmen unter gleichzeitiger Wärmezufuhr ein hochvernetztes Werkstück entsteht.

Die erfindungsgemäßen Beschleuniger können außerdem mit Erfolg in lösungsmittelfreien Systemen auf Basis von Dicyandiamid und Epoxidharzen verwendet werden. Ein typisches Einsatzgebiet ist die Verwendung von heißhärtenden Einkomponentenklebstoffen für die Verklebung von Karosserieteilen in der Automobilindustrie (Bördelnahtkleber).

Herstellung der erforderlichen Beschleuniger

Beispiele

I. Herstellung eines erfindungsgemäßen Beschleunigers aus Isophorondiisocyanat (IPDI), 2-Ethyl-4-methylimidazol (EMI 2,4) und Dimethylamin

111 g IPDI (0,5 Mol) werden in 111 ml Tetrachlorkohlenstoff unter Stickstoff auf ca 40 °C vorgewärmt. Unter Rühren wird eine Lösung von 55 g (EMI 2,4) (0,5 Mol) in 55 ml Tetrachlorkohlenstoff während 1 Stunde zugegeben. Man hält ca. 2 Stunden bei 40 - 50 °C und leitet nach Abkühlen auf RT 22,5 g Dimethylamin (0,5 Mol) ein. Es entsteht eine hochviskose gelbe Lösung, die bei ca. 50 °C im Vakuum vom Lösungsmittel befreit wird. Das Reaktionsprodukt zeigt einen Erweichungspunkt von ca. 82 °C und im IR-Spektrum keine N=C=O-Bande. Es ist löslich in Aceton, Methylethylketon, Methylenchlorid, Tetrachlorkohlenstoff und Propylenglykolmonomethylether.

Ia. Herstellung einer Prepreg-Reaktonsmischung aus I ohne Mitverwendung von Dicyandiamid.

Der erfindungsgemäße Beschleuniger wird bei Raumtemperatur (RT) im Gewichtsverhältnis 1:5 mit Dimethylformamid versetzt. Aus 60 Gew.-Teilen dieser Beschleunigerlösung fertigt man durch Zugabe von 100 Gew.-Teilen eines mittelviskosen EP-Harzes (Epoxi-Äquivalentgewicht ca. 190) eine für die Prepeg-Herstellung verwendbare Imprägniermischung.

Zur Prepregherstellung im Labormaßstab wird ein ca. 0,1 $m^2$ großes Glasfilamentgewebe in Atlasbindung (296 $g/m^2$) mit der Imprägniermischung getränkt und anschließend 5 Minuten bei 100 °C im Umluftofen thermisch behandelt. Es resultieren flexible, aber stark klebrige und daher kaum handhabbare Prepregs mit einem Harzgehalt von ca. 30 Gew.-%, die auch nach mehrtägiger Lagerung zwischen PE-Folien ihre Konsistenz nicht merklich ändern.

Nach einer mindestens 24 stündigen Zwischenlagerung werden jeweils 2 Prepreg-Lagen im Heißpreßverfahren bei 120 °C und 0,1 bar 30 Minuten verpreßt. Das Endprodukt weist eine für mit Glasgewebe verstärkte EP-Harzsysteme äußerst geringe Steifigkeit auf. Die im Torsionsschwingversuch (DIN 53445) ermittelte Übergangstemperatur liegt bei 65 °C.

II. Herstellung eines erfindungsgemäßen Beschleunigers aus Isophorondiisocyanat, 2-Ethyl-4-methylimidazol und Dimethylamin in Dimethylformamid (DMF).

55,5 g IPDI (0,25 Mol) werden in 55,5 g Dimethylformamid unter Stickstoff auf ca. 40 °C vorgewärmt. Unter Rühren wird eine Lösung von 27,5 g EMI 2,4 (0,25 Mol) in 27,5 g Dimethylformamid während 1 Stunde zugegeben. Man hält ca. 2 Stunden bei 40 - 50 °C und leitet nach Abkühlen auf Raumtemperatur 11,25 g Dimethylamin 0,25 Mol) ein.

Das Reaktionsprodukt ist eine ca. 50%ige Lösung in Dimethylformamid und zeigt im IR-Spektrum keine N = C = O-Bande.

IIa. Herstellung einer Prepreg-Reaktionsmischung aus II unter Mitverwendung von Dicyandiamid.

Die Lösung von:

| | |
|---|---|
| 24 g | Dicyandiamid und |
| 21 g | Reaktionsprodukt II in |
| 135 g | Dimethylformamid |

wird mit 60 phr mit einem Epoxidharz (Epoxi-Äquivalentgewicht ca. 190) abgemischt und zur Prepreg-Herstellung eingesetzt. Die Viskosität dieser Imprägnierlösung, gemessen mit einem Kegel-Platte-Rheometer bei 20 °C (Fa. Epprecht Instruments), liegt bei 0,09 Pa. s und ändert sich bei Raumtemperatur-Lagerung auch innerhalb mehrerer Tage nicht meßbar.

Bei der unter Pkt. Ia. beschriebenen Prepreg-Herstellung mit von 5 auf 10 min. geänderter Anhärtezeit resultieren flexible, schwach klebende Produkte, die auch nach mehr als 4-wöchiger Lagerung bei Raumtemperatur zwischen PE-Folien ohne Eigenschaftsverlust zu duromeren Formteilen ausgehärtet werden können. Die im Torsionsschwingversuch nach DIN 53445 ermittelte Übergangstemperatur der ausgehärteten Produkte beträgt 156 °C und dieser Wert verringert sich auch nach 90 Stunden Lagerung der Prüfkörper bei 60 °C nicht.

IIb. Herstellung einer Prepreg-Reaktionsmischung aus II. ohne Mitverwendung von Dicyandiamid.

Der erfindungsgemäße Beschleuniger wird bei Raumtemperatur im Gewichtsverhältnis 2 : 4 mit Dimethylformamid versetzt. Aus 60 Gewichtsteilen dieser Beschleunigerlösung fertigt man durch Zugabe von 100 Gewichtsteilen eines mittelviskosen Epoxidharzes (Epoxid-Äquivalentgewicht ca. 190) eine für die Prepreg-Herstellung verwendbare Imprägniermischung und verarbeitet diese wie in Beispiel Ia. beschrieben. Die im Torsionsschwingversuch (DIN 53 445) ermittelte Übergangstemperatur liegt ebenfalls nur auf dem Niveau von Beispiel Ia.

III. Die Herstellung eines erfindungsgemäßen Beschleunigens aus Butandiol, Isophorondiisocyanat, 2-Ethyl-4-methylimidazol und Dimethylamin.

88,8 g IPDI (0,4 Mol) werden in 97,8 g Dimethylformamid unter Stickstoff auf ca. 75 °C vorgewärmt. 18,0 g Butandiol 1,4 (0,2 Mol) in 18 g Dimethylformamid werden zugetropft und das Reaktionsgemisch ca. 1,5 Stunden bei 75 °C gehalten. Nach Abkühlen auf Raumtemperatur gibt man 22,0 g EMI 2,4 (0,2 Mol) in 22 g Dimethylformamid während 30 Minuten zu. Anschließend leitet man 9,0 g Dimethylamin (0,2 Mol) bei Raumtemperatur ein.

Das Reaktionsgemisch ist eine 50%ige Lösung in Dimethylformamid und zeigt im IR-Spektrum keine N=C=O-Bande.

IIIa. Herstellung eines Prepreg-Reaktionsgemisches aus III unter Mitverwendung von Dicyandiamid

Die Lösung von

| | |
|---|---|
| 24 g | Dicyandiamid und |
| 24 g | Reaktionsprodukt III in |
| 132 g | Dimethylformamid |

wird mit 60 phr mit einem Epoxidharz (Epoxi-Äquivalentgewicht ca. 190) abgemischt und zur Prepreg-Herstellung eingesetzt.

Die mittels Kegel-Platte-Rheometer gemessene Viskosität der Imprägnierlösung beträgt bei 20 °C 0,10 Pa. s. Für die entsprechend Beispiel Ia. hergestellten und nach 4-wöchiger Zwischenlagerung bei Raumtemperatur weiterverarbeiteten (flexiblen, schwach klebenden) Prepregs bestimmt sich im Torsionsschwingversuch DIN 53445 eine Übergangstemperatur von 143 °C.

Zum exakten Vergleich der erfinungsgemäßen Beschleuniger mit den Beschleunigern gemäß dem Stande der Technik wurden Dicyandiamid/Beschleunigerfomulierungen erstellt, bei denen der Gesamtstickstoffgehalt der Beschleunigerkomponenten auf den gleichen Wert berechnet wurde.

IV. Die in Beispiel II beschriebene 50%ige Lösung des Umsetzungsproduktes auf 1 Mol IPDI mit 1 Mol 2-Ethyl-4-methylimidazol und 1 Mol Dimethylamin wird folgendermaßen formuliert:

| | |
|---|---|
| 4,0 g | Dicyandiamid |
| 2,9 g | Lösung II, enthaltend 1,45 g Beschleuniger (18,6 % N) |
| 23,1 g | Dimethylformamid p. a. |

Diese Lösung wird mit 60 phr und einem Epoxidharz (Epoxi-Äquivalentgewicht ca. 190) abgemischt. Prüfergebnisse siehe nachfolende Tabelle.

V. Aus äquivalenten Mengen von Phenylisocyanat und Dimethylamin wird nach bekanntem Verfahren N,N-Dimethyl-N′-phenylharnstoff hergestellt. Das Produkt zeigt nach Umkristallisation aus Wasser einen Schmelzpunkt von 134 °C. Der N-Gehalt beträgt 17,1 %. Aus diesem Produkt wurde folgende Dicyandiamid-Beschleuniger-Lösung hergestellt.

| | |
|---|---|
| 4,0 g | Dicyandiamid |
| 1,5 g | N,N-Dimethyl-N′-phenylharnstoff (17,1 % N) |
| 24,5 g | Dimethylformamid p. a. |

Diese Lösung wird mit 60 phr und einem Epoxidharz (Epoxi-Äquivalentgewicht ca. 190) abgemischt. Prüfergebnisse siehe nachfolende Tabelle.

VI. 111g IPDI (0,5 Mol) werden in 111 ml Aceton p. a. gelöst. Die Lösung wird unter Stickstoff auf 50 - 60 °C erwärmt und mit 110 g 2-Ethyl-4-methylimidazol -(1 Mol) gelöst in 110 ml Aceton p. a. - langsam versetzt. Man hält ca. 3 Stunden bei 50 - 60 °C, so daß im IR-Spektrum keine N=C=O-Bande mehr nachweisbar ist. Nach Abziehen des Acetons erhält man einen Feststoff. Der N-Gehaltbeträgt 19,0 %.

Aus diesem Produkt wird folgende Dicyandiamid-Beschleuniger-Lösung hergestellt:

| | |
|---|---|
| 4,0 g | Dicyandiamid |
| 1,35 g | des Produktes VI |
| 24,65 g | Dimethylformamid p. a. |

Diese Lösung wird mit 60 phr und einem Epoxidharz (Epoxi-Äquivalentgewicht ca. 190) abgemischt. Prüfergebnisse siehe nachfolgende Tabelle.

Die in der nachstehenden Tabelle enthaltenen Viskositäten der Imprägnierlösungen wurden bei 20 °C in einem Kegel-Platte-Rheometer, wie oben angegeben, ermittelt, wobei die Lösungen zwischen der 1. und 2. Messung 6 Tage in einem verschlossenen Behältnis bei Raumtemperatur gelagert wurden.

Die ebenfalls in der Tabelle enthaltenen Anspringtemperaturen wurden mittels DSC (Gerät TA 3000 Fa. Mettler) an frisch gefertigten Imprägnierlösungen bei einer Starttemperatur von 10 °C und einer Aufheizrate von 20 °C/min ermittelt. Die angegebene Reaktionsdauer der verschiedenen Imprägnierlösungen entstammt der kinetischen Analyse, die an DSC-Messungen dieser Produkte bei einer Aufheizrate von 1 °C/min. durchgeführt wurde.

Die Übergangstemperaturen wurden im Torsionsschwingversuch nach DIN 53445 mit einer Aufheizrate von 1 °C/min. ermittelt, wobei die Probekörper, wie in Beispiel Ia. beschrieben, zunächst geprepregt und anschließend zum Duromer ausgehärtet wurden.

| Materialkenngrößen bei gleichen den Gesamtstickstoffgehalt berücksichtigenden Beschleunigermengen | | | | | | | |
|---|---|---|---|---|---|---|---|
| Beispiel | Patentsituation | Viskosität der Imrägnierlösung/20 °C | | Anstieg | Anspringtemp. | Reaktionsdauer bei 120°C | Übergangstemperatur lt. Torsionsschwingversuch (DIN 53445) |
| | | bei d. Herstell. | nach 6 Tagen RT-Lagerung | | nach DSC-Messung | | |
| IV | erfindungsgemäß | 80 m Pa. s | 80 m Pa. s | 0 % | 132 °C | 23 min. | 151 ° C |
| V | nicht erfindungsgemäß | 70 m Pa. s | 70 m Pa. s | 0 % | 135 °C | 22,4 min. | 137 °C |
| VI | nicht erfindungsgemäß | 80 m Pa. s | 150 m Pa. s | 88 % | 98 °C | 15 min. | 152 °C |

**Ansprüche**

1. Verbindungen der allgemeinen Formel (I),

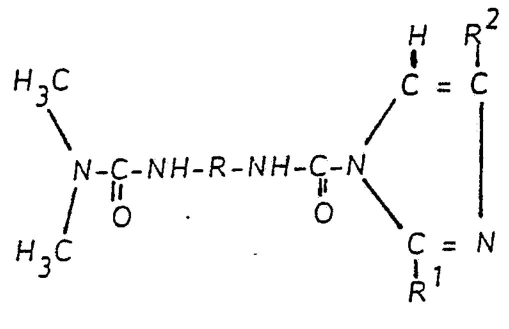

(I)

worin R ein gegebenenfalls substituierter, aliphatischer, cycloaliphatischer, aromatischer, araliphatischer Rest ist und $R^1$ und $R^2$ unabhängig voneinander die Bedeutung H, $-CH_3$, $-C_2H_5$, $-C_6H_5$ haben können.

2. Verbindungen gemäß Anspruch 1, worin R der Kohlenwasserstoffrest des Isophorondiisocyanats $R^1 = C_2H_5$ und $R^2 = CH_3$ ist.

3. Verbindungen gemäß Anspruch 1, in welchem R ein gegebenenfalls substituierter, aliphatischer, cycloaliphatischer, aromatischer, araliphatischer Kohlenwasserstoffrest ist, der ein oder mehrere Urethan- und/oder Harnstoffgruppen enthält.

4. Verbindungen gemäß Anspruch 1, worin R der Rest eines Adduktes aus Isophorondiisocyanat und Butandiol im Molverhältnis 2 : 1 ist.

5. Verwendung von Verbindung der allgemeinen Formel (I),

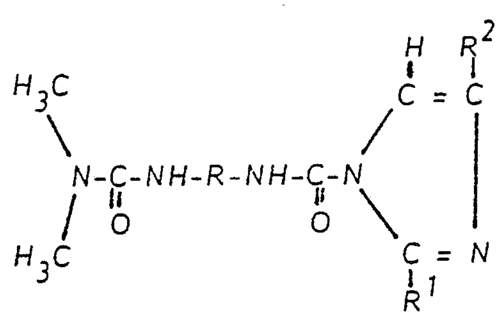

(I)

worin R ein gegebenenfalls substituierter, ggf. Harnstoff- und/oder Urethangruppen enthaltender, aliphatischer, cycloaliphatischer, aromatischer, araliphatischer Rest ist und $R^1$ und $R^2$ unabhängig voneinander die Bedeutung H, $-CH_3$, $-C_2H_5$, $-C_6H_5$ haben können als Beschleuniger in härtbaren Mischungen aus

a) Epoxidharz
b) Dicyandiamid und ggf.
c) Lösungsmitteln, Füllstoffen, Verstärkung- oder Einlagematerialien, Pigmenten, Hilfsstoffen.

6. Härtbare Epoxidharz-Zusammensetzungen enthaltend

a) ein Epoxidharz mit durchschnittlich mehr als einer Epoxidgruppe pro Molekül,
b) Dicyandiamid und ggf.
c) Lösungsmittel, Füllstoffe, Verstärkungs- oder Einlagematerialien, Pigmente, Hilfsstoffe und
d) als Härtungsbeschleuniger Verbindungen nach Anspruch 1 bis 4.

7. Härtbare Epoxidharzusammensetzungen gemäß Anspruch 6, in der die Menge an Härtungsbeschleuniger 0,1 bis 10 Gew.-Teile, bezogen auf 100 Gew.-Teile Epoxidharz, beträgt.

8. Härtbare Mischungen gemäß Anspruch 6, in der als Epoxidharzkomponente a) Glycidylether auf Basis von Bisphenol A mit Epoxidwerten von 0,2 bis 0,6 verwendet werden.

9. Härtbare Mischungen gemäß Ansprüchen 6 bis 8, dadurch gekennzeichnet, daß die Verstärkungsmaterialien bei Raumtemperatur mit dem Bindemittel, bestehend aus
a) Epoxidharz
b) Dicyandiamid
c) Lösungsmittel
d) einem Härtungsbeschleuniger gemäß Ansprüchen 1 bis 4,
imprägniert und in den halbfesten, aber noch schmelzbaren Zustand (B-Zustand) überführt werden.

10. Epoxidharzformkörper, dadurch gekennzeichnet, daß in erster Stufe die Verstärkungsmaterialien bei Raumtemperatur mit dem Bindemittel, bestehend aus
a) Epoxidharz
b) Dicyandiamid
c) Lösungsmittel
d) einem Härtungsbeschleuniger gemäß Ansprüchen 1 bis 4,
imprägniert und in den halbfesten, aber noch schmelzbaren Zustand (B-Zustand) überführt werden und in zweiter Stufe die so hergestellten Prepregs allein und unter Formgebung oder zwischen zu verklebende Substrate gebracht, unter Anwendung von Druck bei erhöhter Temperatur ausgehärtet werden.

Patentansprüche für folgenden Vertragsstaat: ES

1. Verfahren zur Herstellung von Verbindungen der allgemeinen Formel (1).

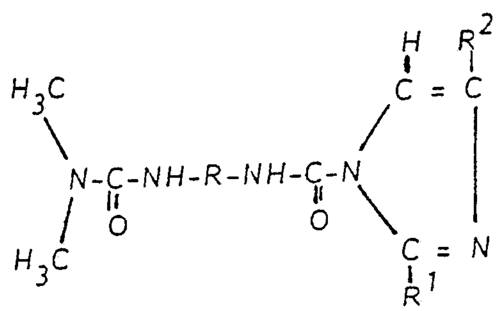

(I)

worin R ein gegebenenfalls substituierter, aliphatischer, cyclcaliphatischer, aromatischer, araliphatischer Rest ist und $R^1$ und $R^2$ unabhängig voneinander die Bedeutung H, $-CH_3$, $-C_2H_5$, $-C_6H_5$ haben können, durch Umsetzung der entsprechenden aliphatischen, cycloaliphatischen, aromatischen oder araliphatischen Diisocyanate mit den entsprechenden Imidazolen und Dimethylamin.

2. Verfahren zur Herstellung von Verbindungen gemäß Anspruch 1, worin R der Kohlenwasserstoffrest des Isophorondiisocyanats $R^1 = C_2H_5$ und $R^2 = CH_3$ ist.

3. Verfahren zur Herstellung von Verbindungen gemäß Anspruch 1, in welchem R ein gegebenenfalls substituierter, aliphatischer, cycloaliphatischer, aromatischer, araliphatischer Kohlenwasserstoffrest ist, der ein oder mehrere Urethan- und/oder Harnstoffgruppen enthält.

4. Verfahren zur Herstellung von Verbindungen gemäß Anspruch 1, worin R der Rest eines Adduktes aus Isophorondiisocyanat und Butandiol im Molverhältnis 2 : 1 ist.

5. Verwendung von Verbindungen der allgemeinen Formel (I),

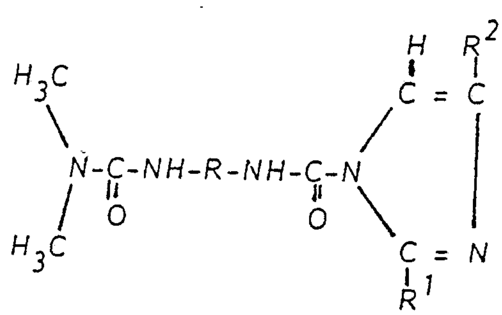

(I)

worin R ein gegebenenfalls substituierter, gegebenenfalls Harnstoff- und/oder Urethangruppen enthaltender, aliphatischer, cycloaliphatischer, aromatischer, araliphatischer Rest ist und $R^1$ und $R^2$ unabhängig voneinander die Bedeutung H, $-CH_3$, $-C_2H_5$, $-C_6H_5$ haben können als Beschleuniger in härtbaren Mischungen aus

a) Epoxidharz
b) Dicyandiamid und gegebenenfalls
c) Lösungsmitteln, Füllstoffen, Verstärkungs- oder Einlagematerialien, Pigmenten, Hilfsstoffen.

6. Härtbare Epoxidharz-Zusammensetzungen enthaltend
a) ein Epoxidharz mit durchschnittlich mehr als einer Epoxidgruppe pro Molekül,
b) Dicyandiamid und gegebenenfalls
c) Lösungsmittel, Füllstoffe, Verstärkungs- oder Einlagematerialien, Pigmente, Hilfsstoffe und
d) als Härtungsbeschleuniger Verbindungen nach Anspruch 1 bis 4.

7. Härtbare Epoxidharzusammensetzungen gemäß Anspruch 6, in der die Menge an Härtungsbeschleuniger 0,1 bis 10 Gew.-Teile, bezogen auf 100 Gew.-Teile Epoxidharz, beträgt.

8. Härtbare Mischungen gemäß Anspruch 6, in der als Epoxidharzkomponente a) Glycidylether auf Basis von Bisphenol A mit Epoxidwerten von 0,2 bis 0,6 verwendet werden.

9. Härtbare Mischungen gemäß Ansprüchen 6 bis 8, dadurch gekennzeichnet, daß die Verstärkungsmaterialien bei Raumtemperatur mit dem Bindemittel, bestehend aus
a) Epoxidharz
b) Dicyandiamid
c) Lösungsmittel
d) einem Härtungsbeschleuniger gemäß Ansprüchen 1 bis 4, imprägniert und in den halbfesten, aber noch schmelzbaren Zustand (B-Zustand) überführt werden.

10. Verfahren zur Herstellung von Epoxidharzformkörper, dadurch gekennzeichnet, daß in erster Stufe die Verstärkungsmaterialien bei Raumtemperatur mit dem Bindemittel, bestehend aus
a) Epoxidharz
b) Dicyandiamid
c) Lösungsmittel
d) einem Härtungsbeschleuniger gemäß Ansprüchen 1 bis 4, imprägniert und in den halbfesten, aber noch schmelz baren Zustand (B-Zustand) überführt werden und in zweiter Stufe die so hergestellten Prepregs allein und unter Formgebung oder zwischen zu verklebende Substrate gebracht, unter Anwendung von Druck bei erhöhter Temperatur ausgehärtet werden.

Europäisches Patentamt

# EUROPÄISCHER RECHERCHENBERICHT

Nummer der Anmeldung

EP 89 10 7725

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | US-A-3 717 612 (E.P. BABAYAN)<br>* Ansprüche; Beispiele 4,5 *<br>--- | 1-10 | C 07 D 233/61<br>C 08 G 18/58<br>C 08 G 18/80<br>C 08 G 59/40<br>C 08 G 59/68 |
| D,A | US-A-4 335 228 (B.D. BEITCHMAN)<br>* Ansprüche *<br>----- | 1-10 | |
| | | | **RECHERCHIERTE SACHGEBIETE (Int. Cl.4)** |
| | | | C 07 D 233/00<br>C 08 G 18/00<br>C 08 G 59/00 |

Der vorliegende Recherchenbericht wurde für alle Patentansprüche erstellt

| Recherchenort | Abschlußdatum der Recherche | Prüfer |
|---|---|---|
| DEN HAAG | 06-09-1989 | WRIGHT M.W. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)